# FASCICULE DE BREVET EUROPEEN

(11) **EP 0 859 635 B1**
(45) Date de publication et mention de la délivrance du brevet: **21.04.1999**
(21) Numéro de dépôt: 96934972.9
(22) Date de dépôt: 25.10.1996
(51) Int. Cl.: A61K 47/12, A61K 31/135

(54) **COMPOSITIONS PHARMACEUTIQUES STABILISEES CONTENANT DE LA DOBUTAMINE**
STABILISIERTE PHARMAZEUTISCHE ZUSAMMENSETZUNGEN ENTHALTEND DOBUTAMINE
STABILISED PHARMACEUTICAL COMPOSITIONS CONTAINING DOBUTAMINE

(30) Priorité: 27.10.1995 FR 9512688
(43) Date de publication de la demande: 26.08.1998
(73) Titulaire: RHONE-POULENC RORER S.A., 92160 Antony (FR)
(72) Inventeur: KALIFA-MADAR, Danièle, F-91190 GIR SUR YVETTE (FR); DIETLIN, François, F-78230 Le Pecq (FR)
(74) Mandataire: Le Pennec, Magali
(86) Numéro de dépôt international: FR9601671
(87) Numéro de publication internationale: WO9715329

(56) Documents cités:
- EP-A- 0 187 019
- WO-A-94/13274
- FR-A- 2 231 377
- US-A- 3 808 317
- ACTA FAC. PHARM. UNIV., vol. 36, 1981, COMENIANAE, pages 173-191, XP000576849 SPRINGER V. ET AL: "Stabilization of eye drops containing adrenaline"
- PHARM. ACT. HELV., vol. 61, no. 2, 1986, pages 34-41, XP000576693 THOMA K. ET AL: "Stabilization of adrenaline solutions. 2. Report on the stability of adrenaline solutions"
- SUCKER H. ET AL: "Pharmazeutische Technologie" , THIEME VERLAG , 1991 XP002009773 189560 voir page 215; tableau 5.18
- AM. J. HOSP. PHARM., vol. 39, no. 11, 1982, pages 1923-1925, XP000576806 PILTZ G. ET AL: "Stability of Dobutamine Hydrochloride in selected large volume parenterals"

## Description

La présente invention concerne de nouvelles formulations liquides à base de dobutamine.

Il est connu, selon l'article de Bishara R. H. et Long H. B. Dobutamine hydrochloride paru dans Analytical Profiles of drug Substances, Academic Press (1979), volume 8, pages 139-158, que les solutions de dobutamine dans l'eau ne sont pas stables d'une part lors de la stérilisation par la chaleur et d'autre part au cours de leur conservation.

La dégradation de la dobutamine apparaît sous forme d'une coloration rose qui vire progressivement au gris et peut même se traduire par un précipité. Les produits de dégradation sont des dérivés qui ne sont pas à ce jour identifiés. Seule la couleur de la solution permet de déceler leur présence.

Pour résoudre ce problème, il est connu depuis longtemps de stabiliser lesdites solutions en ajoutant du sulfite. Ainsi la dobutamine est actuellement commercialisée en solution liquide prête à l'emploi selon la composition suivante :

| | |
|---|---|
| • dobutamine HCl | 280,2 mg |
| • métabisulfite de sodium | 4,5 mg |
| • HCl ou NaOH q.s.p | pH 4,0 |
| • eau q.s.p | 20 ml |

Ces solutions sont ensuite conditionnées sous azote pour prévenir au maximum l'oxydation de la dobutamine lors de sa conservation.

Il est connu que les sulfites posent, chez l'homme, des problèmes de pharmacovigilance et de toxicité.

L'industrie pharmaceutique cherche donc depuis longtemps à préparer des solutions de dobutamine prêtes à l'emploi, présentant des critères de stabilité suffisants pour permettre la stérilisation desdites solutions par la chaleur et/ou leur conservation pendant des périodes de plusieurs mois mais exemptes de sulfites.

La présente invention a atteint cet objectif et concerne des compositions pharmaceutiques liquides contenant de la dobutamine et de l'acide ascorbique ou un de ses dérivés en quantité suffisante pour préserver sa stabilité.

La composition pharmaceutique liquide est de préférence une composition injectable aqueuse. Le dérivé de l'acide ascorbique préféré qui est utilisé dans ces compositions est l'ascorbate de sodium.

La quantité d'acide ascorbique ou de dérivé de l'acide ascorbique qui est utilisée en poids est de préférence supérieure à 1 % par rapport à la dobutamine et encore plus préférentiellement comprise entre 1,5 et 90 %. Il est évident que des quantités supérieures pourraient être utilisées puisque ce dérivé n'est pas toxique pour l'homme, mais dans la mesure où l'acide ascorbique ou son dérivé sont utilisés pour stabiliser la dobutamine, des quantités suffisantes pour maintenir la stabilité sont préférées.

Ainsi, des quantités pondérales encore plus limitées et situées ente 2 et 20 % d'acide ascorbique ou d'un de ses dérivés pour 100 g de dobutamine sont suffisantes pour réaliser cet objectif, on préfère tout particulièrement utiliser des quantités pondérales comprises entre 3,5 et 10 %.

La dobutamine et l'acide ascorbique ou son dérivé présentant des stabilités variables avec la nature du pH, il est recommandé de maintenir le pH de la solution sous forme acide. Ainsi, un pH de ladite solution compris entre 4 et 7 est préféré. En dessous de pH 4, les solutions sont difficilement utilisables pour une utilisation par voie injectable. Au dessus de pH 7, la dobutamine n'est pas stable. Il est ainsi clairement exprimé dans les conditions de préparation telles que décrites par exemple dans le dictionnaire VIDAL de ne pas mélanger les solutions de dobutamine avec des solutions de bicarbonate de sodium à 5 % ou des solutions fortement alcalines.

Les solutions préférées selon l'invention seront fixées à un pH voisin de 5,5. Dans le cas où le pH est fixé aux environs de 5,5 des quantités pondérales d'ascorbate de sodium calculées par rapport à la dobutamine comprises entre 1 et 3 % sont suffisantes pour stabiliser la dobutamine lors de la stérilisation. Il est ainsi avantageux d'ajouter à la solution de dobutamine et d'acide ascorbique ou d'un de ses dérivés un agent de stabilisation du pH tel que le phosphate monoacide de sodium.

La composition ainsi préparée pourra être stérilisée par traitement thermique, par exemple à 120°C pendant quelques minutes ou par filtration stérilisante.

La composition préférée selon l'invention a la composition suivante :

| | |
|---|---|
| dobutamine chlorhydrate | 280,2 mg |
| Na₂HPO₄ | 150 mg |
| Ascorbate de sodium | 25 mg |
| HCl IN q.s.p | pH 5,5 |
| eau q.s.p | 20 ml |

### 1^{er} ESSAI COMPARATIF

### Protocole

Le premier essai a été effectué avec une composition identique à celle de Dobutrex® mais sans sulfite :

| | |
|---|---|
| Dobutamine, HCl | 280,2 mg |
| HCl ou NaOH Q.S.P | pH 4,0 |
| eau pour préparation injectable. Q.S.P | 20 ml |
| azote Q.S. | |

### Résultats :

Le produit se dégrade à la chaleur. Il se forme un précipité gris durant la stérilisation.

### 2^{ème} ESSAI COMPARATIF

### Protocole :

La composition est identique à celle de Dobutrex® mais sans sulfite ; la solution est stérilisée par filtration.

| | |
|---|---|
| Dobutamine, HCl | 280,2 mg |
| HCl ou NaOH Q.S.P | pH 4,0 |
| eau pour préparation injectable Q.S.P | 20 ml |
| azote Q.S. | |

### Résultats :

Le produit n'est pas stable. Juste après la fabrication, la teneur en dobutamine, HCl est de 268 mg/20 ml pour une limite inférieure de 266 mg/20 ml. Après 1 mois de conservation à une température ambiante, il apparaît une coloration rose ou un précipité gris.

### 3^{ème} ESSAI

### But :

Fabriquer une solution avec sulfite, stérilisée par filtration ou par la chaleur.

### Protocole :

La composition est identique à celle du Dobutrex® :

| | |
|---|---|
| Dobutamine, HCl | 280,2 mg |
| Métabisulfite de sodium | 4,5 mg |
| HCl ou NaOH Q.S.P | pH 4,0 |
| eau pour préparation injectable Q.S.P azote Q.S. | 20 ml |

Une partie du lot a été autoclavée à 121°C pendant 20 minutes (277 flacons) ce qui a donné le lot A. L'autre partie non autoclavée (592 flacons) a constitué le lot B. Les lots sont conservés à 25°C, 40°C et 50°C.

### Résultats :

Après fabrication, le lot A présente une légère coloration jaune pâle. Les deux lots ne sont pas stables ; les sulfites sont consommées durant la fabrication et la conservation, comme le montrent les valeurs présentées dans le tableau ci-après.

| PRODUIT | TEMPS/TEMPERATURE | POURCENTAGE DE SO₂ |
|---|---|---|
| Dobutrex - Lot commercial | | 2,56 |
| Essai A | Temps O | 1,16 |
| | 1 mois/25°C | 0,49 |
| | 1 mois/40°C | 0,00 |
| | 1 mois/50°C | 0,00 |
| Essai B | Temps O | 1,60 |
| | 1 mois/25°C | 0,70 |
| | 1 mois/40°C | 0,40 |
| | 1 mois/50°C | 0,00 |

Pour les deux lots, après 3 mois de conservation à 25°C, les solutions présentent une coloration rose ou un précipité gris. La teneur en Dobutamine reste cependant dans les limites de ± 5 pour cent de la valeur théorique.

### 1^{er} ESSAI SELON L'INVENTION

Solutions avec de l'ascorbate de sodium en faisant varier le pH.

### Protocole :

La solution est préparée en joutant une quantité d'ascorbate de sodium (19 ml d'une solution à 0,05 - 0,10 ou 0,15 pour cent, pour 20 ml de produit fini) et en faisant varier le pH (3,5 - 4,0 ou 4,5). Il n'y a pas de sulfite.

Les solutions sont préparées sous azote et autoclavées à 121°C/20 minutes.

Les solutions sont analysées après stérilisation et elles sont conservées à 50°C.

### Résultats :

### Immédiatement après stérilisation par la chaleur :

- il n'y a pas de dégradation de la dobutamine à la chaleur,
- la profil chromatographique révèle des pics secondaires dont l'importance augmente avec la quantité d'ascorbate apportée, pour une même valeur de pH. L'importance de ces pics diminue de pH 3,5 à 4,5,
- la coloration jaune augmente avec la quantité d'ascorbate apportée, pour un même pH. Cette coloration diminue de pH 3,5 à 4,5.

### Au bout de 1 mois et demi à 50°C :

Les valeurs sont regroupées dans le tableau ci-après.

| Concentration en ascorbate dans la solution finale | pH initial | Absorbance à 308 nm* | Dobutamine (% de la valeur initiale) | % des pics secondaires (rapport de surfaces) |
|---|---|---|---|---|
| 0,1425 % | 3,5 | 2,6910 | 98 | 4,68 |
| | 4,0 | 1,1669 | 100 | 2,47 |
| | 4,5 | 0,6230 | 102 | 0,43 |
| 0,095 % | 3,5 | 2,4669 | 98 | 2,44 |
| | 4,0 | 0,9400 | 99 | 1,36 |
| | 4,5 | 0,8960 | 96 | 0,78 |
| 0,0475 % | 3,5 | Trouble | 103 | 2,29 |
| | 4,0 | Trouble | 103 | 0,93 |
| | 4,5 | Trouble | 103 | 0,24 |

| | | | | |
|---|---|---|---|---|
| * de la solution contre la solution non autoclavée. | | | | |

Les solutions fabriquées avec 0,0475 pour cent d'ascorbate ne sont pas stables. Le pH 4,5 donne les meilleurs résultats. Avec ce pH, la coloration et les pics secondaires sont les plus faibles

### 2^{eme} ESSAI SELON L'INVENTION

La variation de la quantité d'ascorbate ajouté a été étudiée à un pH fixé à 4,5.

On prépare des solutions avec les concentrations en ascorbate de sodium suivantes : 45 ; 54 ; 63 ; 72 ; 81 ; 90 ; 135 ; 271 mg pour 100 ml de solution finale à partir de 19 ml de solutions d'ascrobate de sodium aux concentrations suivantes : 48 ; 57 ; 67 ; 76 ; 86 ; 95 ; 142 ; 285 mg pour 100 ml qui sont complétées à 20 ml.

Les solutions sont préparées sous azote et autoclavées à 120°C/20 minutes.

Les solutions sont analysées après stérilisation.

| Concentration en ascorbate dans la solution % | pH après stérilisation | Absorbance à 308 nm* | teneur en dobutamine (g/100ml) | somme des pics secondaires |
|---|---|---|---|---|
| 0,045 | 4,9 | 0,618 | 1,43 | 0,98 |
| 0,054 | 4,9 | 0,471 | 1,46 | 0,63 |
| 0,063 | 4,7 | 0,584 | 1,47 | 0,74 |
| 0,072 | 4,7 | 0,569 | 1,47 | 0,70 |
| 0,081 | 4,7 | 0,594 | 1,46 | 0,72 |
| 0,090 | 4,7 | 0,710 | 1,48 | 0,92 |
| 0,135 | 4,6 | 0,804 | 1,50 | 0,95 |
| 0,271 | 4,6 | 1,136 | 1,44 | 1,46 |

| | | | | |
|---|---|---|---|---|
| * de la solution contre la solution non autoclavée | | | | |

La concentration minimale en ascorbate de sodium à pH 4,5 qui protège la dobutamine est d'environ 54 mg/100 ml ce qui correspond à un rapport pondéral par rapport à la dobutamine d'environ 3,8 %.

Les solutions non stérilisées et maintenues à l'air sont roses en 24 heures, sauf pour les concentrations en ascorbate de 135 et 271 mg/100 ml. La concentration de 135 mg/100 ml est la concentration minimale qui permet de protéger la dobutamine pendant 24 heures après ouverture du flacon, la solution restant à l'air libre. Cette concentration correspond à un rapport pondéral par rapport à la dobutamine de 9,6 %.

### 3^{ème} ESSAI SELON L'INVENTION

### But :

Optimiser la stabilité en faisant varier le pH

### Protocole :

Les solutions sont toutes fabriquées avec une concentration en ascorbate de sodium pour 100 ml de solution de 0,135 %.

Le pH est ajusté à 4,5 - 5,0 - 5,5 - 6,0 - 6,5 - 7,0 par de l'acide chlorhydrique.

Il n'y a pas de sulfite.

La fabrication est faite sous azote.

Les flacons sont stérilisés à 120°C/20 minutes.

### Résultats :

### Immédiatement après stérilisation par la chaleur :

- la dobutamine est stable à la chaleur,
- la coloration diminue de pH 4,5 à 5,5 et augmente ensuite avec le pH.
   les pics secondaires (provenant de l'ascorbate) diminuent de pH 4,5 à 5,5.

| pH initial | Absorbance à 308 nm* | Dobutamine, HCl (g/100ml) | Somme des pics secondaires |
|---|---|---|---|
| 4,5 | 0,823 | 1,46 | 1,19 |
| 5,0 | 0,691 | 1,44 | 0,56 |
| 5,5 | 0,644 | 1,45 | 0,37 |
| 6,0 | 0,741 | 1,45 | 0,35 |
| 6,5 | 0,762 | 1,43 | 0,36 |
| 7,0 | 0,895 | 1,51 | 0,33 |

| | | | |
|---|---|---|---|
| * de la solution diluée au ½ contre de l'eau | | | |

Une absorbance minimale de la solution est obtenue avec un pH de 5,5. La teneur en dobutamine n'est pas affectée par la variation de pH dans la fourchette étudiée.

### Après un mois à 50°C

- la dobutamine est stable,
- les pics secondaires (provenant de l'ascorbate) diminuent de pH 4,5 à 5,5. A pH 5,5 leur somme est de 0,11 pour cent.

| pH initial | Dobutamine (pourcentage de la teneur initiale) | Pourcentage des pics secondaires | Absorbance à 308 nm* |
|---|---|---|---|
| 4,5 | 100 | 0,97 | 1,033 |
| 5,0 | 100 | 0,32 | 0,572 |
| 5,5 | 99 | 0,11 | 0,313 |

| | | | |
|---|---|---|---|
| * de la solution diluée au ½ contre de l'eau. | | | |

### 4^{ème} ESSAI SELON L'INVENTION

But : optimiser la quantité minimale d'ascorbate de sodium en fonction du pH.

### Protocole opératoire

| | | | | | | | | |
|---|---|---|---|---|---|---|---|---|
| dobutamine, HCl | 1,4 | 1,4 | 1,4 | 1,4 | 1,4 | 1,4 | 1,4 | 1,4 |
| Ascorbate de Na (0,0480 g/100 ml) | 95 ml | 95 ml | | | | | | |
| Ascorbate de Na (0,0383 g/100 ml) | | | 95 ml | 95 ml | | | | |
| Ascorbate de Na (0,0292 g/100 ml) | | | | | 95 ml | 95 ml | | |
| Ascorbate de Na (0,0106 g/100 ml) | | | | | | | 95 ml | 95 ml |
| HCl 0,1 N ou NaOH 0,1N QSP | pH5,5 | pH6,5 | pH5,5 | pH6,5 | pH5,5 | pH6,5 | pH5,5 | pH6,5 |
| H₂O QSP | 100ml | 100ml | 100ml | 100ml | 100ml | 100ml | 100ml | 100ml |
| Préparations stérilisées sous azote 120°C/20mn | | | | | | | | |

### Résultats

| Concentration en ascorbate de sodium (g/100 ml) | pH avant stérilisation | pH après stérilisation | Absorbance à 308 nm* | teneur en Dobutamine HCl | Somme des pics secondaires |
|---|---|---|---|---|---|
| 0,0456 | 5,5 | 5,9 | 0,383 | 1,4 | 0,06 |
| | 6,5 | 6,4 | 0,405 | 1,4 | 0,03 |
| 0,0364 | 5,5 | 5,8 | 0,326 | Pas de variation significative | 0,04 |
| | 6,5 | 6,3 | 0,355 | | 0,03 |
| 0,0277 | 5,5 | 5,7 | 0,238 | | 0,05 |
| | 6,5 | 6,3 | 0,317 | | 0,02 |
| 0,0101 | 5,5 | 5,8 | 0,252 | | 0,02 |
| | 6,5 | 6,4 | 0,340 | | 0,03 |
| 0 | 5,5 | 3,99 | 0,635 | non déterminée | |
| | 6,5 | 6,21 | 0,909 | | |

| | | | | | |
|---|---|---|---|---|---|
| * de la solution diluée au demi contre de l'eau | | | | | |

### 5ème ESSAI SELON L'INVENTION

### But :

Stabiliser le pH.

### Protocole :

Compte tenu des résultats obtenus à l'essai 3 selon l'invention, une valeur de pH 5,5 est retenue. Pour stabiliser le pH, on ajoute du phosphate disodique. La quantité optimale est déterminée en jugeant de l'effet tampon de solutions à concentrations variées en phosphate disodique.

### Résultats :

L'effet tampon est maximal à une concentration de 150 mg/20 ml

### 6^{ème} ESSAI SELON L'INVENTION

### But :

Prévoir l'importance de la formation de pics secondaires durant la conservation, en augmentant la teneur en ascorbate. On peut envisager le fait que les solutions moins concentrées présenteront à long terme des pics secondaires dont l'importance sera au plus égale à celle déterminée lors de cet essai.

### Protocole :

La solution est préparée selon la formulation suivante :

| | |
|---|---|
| Dobutamine, HCl | 280,2 mg |
| NaHPO₄, 2H₂O | 150 mg |
| Ascorbate de sodium | 250 mg (rapport pondéral/dobutamine=89 %) |
| HCl 1N Q.S.P | pH 5,5 |
| Eau pour préparation injectable Q.S.P | 20 ml |
| azote Q.S. | |

L'ascorbate est apporté à une concentration environ 10 fois supérieure à celle retenue pour l'essai 3. La solution est conservée à 25°C et 40°C pendant 6 mois.

Une partie des solutions a été stérilisée par la chaleur :
121°C/20 minutes
124°C/10 minutes
125°C/8 minutes
126°C/6 minutes

### Résultats :

Toutes les solutions stérilisées par la chaleur sont jaunes mais la teneur en dobutamine reste inchangée.

Pour les solutions non autoclavées :
- la dobutamine est stable à 25°C mais elle diminue à 40°C,
- l'acide ascorbique est stable à 25°C mais il diminue à 40°C
- le phosphate disodique permet de stabiliser le pH,
- la coloration n'augmente qu'à 40°C,
- après 6 mois les pics secondaires (provenant de l'ascorbate) représentent 0,79 pour cent à 25°C et 2,14 pour cent à 40°C.

Les résultats après 6 mois de conservation sont regroupés dans le tableau ci-après.

| Températures | pH | Dobutamine (% teneur initiale) | % des pics secondaires | Teneur en Absorbance ascorbate à 308 nm* (%) | |
|---|---|---|---|---|---|
| 25°C | 5,5 | 100 | 0,79 | 1,13 | 2,53 |
| 40°C | 5,7 | 100 | 2,14 | 0,98 | 2,99 |

| | | | | | |
|---|---|---|---|---|---|
| * de la solution diluée au ½ contre de l'eau | | | | | |

La formulation suivante peut être retenue :

| | |
|---|---|
| Dobutamine, HCl | 280,2 mg |
| NaHPO₄, 2H₂O | 150 mg |
| Ascorbate de sodium | 27 mg |
| HCl 1N Q.S.P | pH 5,5 |
| Eau pour préparation injectable Q.S.P | 20 ml |
| azote Q.S. | |

Immédiatement avant la stérilisation l'absorbance de la solution (dilution au demi contre de l'eau) à 308 nm est de 0,54. Après stérilisation elle est de 0,64. Cette augmentation faible montre la bonne stabilité de la préparation à la chaleur. Il est toutefois possible de se dispenser d'une stérilisation par la chaleur, au profit d'une préparation dans des conditions aseptiques avec double filtration stérilisante.

## Revendications

1. Compositions pharmaceutiques liquides et stables contenant de la dobutamine et de l'acide ascorbique ou un de ses dérivés en quantité suffisante pour préserver sa stabilité.

2. Compositions pharmaceutiques selon la revendication 1 caractérisées en ce que la quantité d'acide ascorbique utilisée par rapport à la dobutamine est supérieure à 1 % en poids.

3. Compositions pharmaceutiques selon la revendication 2 caractérisées en ce que la quantité d'acide ascorbique utilisée par rapport à la dobutamine est comprise entre 1,5 et 90 % en poids et de préférence entre 2 et 20 % en poids et encore plus préférentiellement entre 3,5 et 10 %.

4. Compositions pharmaceutiques selon l'une quelconque des revendications précédentes caractérisées en ce que le pH de la solution est ajusté entre 4 et 7 et de préférence à environ 5,5.

5. Compositions selon la revendication 4 caractérisées en ce que, lorque la quantité d'acide ascorbique utilisée par rapport à la dobutamine est comprise entre 1 et 3 % en poids, le pH est fixé à environ 5,5.

6. Compositions pharmaceutiques selon l'une quelconque des revendications précédentes répondant à la formule suivante :
| | |
|---|---|
| Dobutamine chlorhydrate | 280,2 mg |
| Na₂HPO₄ | 150 mg |
| Ascorbate de sodium | 27 mg |
| HCl IN q.s.p | pH 5,5 |
| eau q.s.p | 20 ml |

## Claims

1. Liquid and stable pharmaceutical compositions containing dobutamine and ascorbic acid or one of its derivatives in a quantity sufficient to preserve the stability thereof.

2. Pharmaceutical compositions according to Claim 1, characterized in that the quantity of ascorbic acid used relative to dobutamine is greater than 1% by weight.

3. Pharmaceutical compositions according to Claim 2, characterized in that the quantity of ascorbic acid used relative to dobutamine is between 1.5 and 90% by weight, and preferably between 2 and 20% by weight, and still more preferably between 3.5 and 10%.

4. Pharmaceutical compositions according to any one of the preceding claims, characterized in that the pH of the solution is adjusted to between 4 and 7, and preferably to about 5.5.

5. Compositions according to Claim 4, characterized in that when the quantity of ascorbic acid used relative to dobutamine is between 1 and 3% by weight, the pH is set at about 5.5.

6. Pharmaceutical compositions according to any one of the preceding claims, corresponding to the following formula:
| | |
|---|---|
| Dobutamine hydrochloride | 280.2 mg |
| Na₂HPO₄ | 150 mg |
| Sodium ascorbate | 27 mg |
| HCl IN qs | pH 5.5 |
| Water qs | 20 ml |

## Patentansprüche

1. Flüssige und stabile pharmazeutische Zusammensetzungen, enthaltend Dobutamin und Ascorbinsäure oder eines ihrer Derivate in ausreichender Menge, um dessen Stabilität zu gewährleisten.

2. Pharmazeutische Zusammensetzungen gemäß Anspruch 1, dadurch gekennzeichnet, daß die in Bezug auf Dobutamin verwendete Menge an Ascorbinsäure mehr als 1 Gew.% beträgt.

3. Pharmazeutische Zusammensetzungen gemäß Anspruch 2, dadurch gekennzeichnet, daß die in Bezug auf Dobutamin verwendete Menge an Ascorbinsäure zwischen 1,5 und 90 Gew.%, bevorzugt zwischen 2 und 20 Gew.% und insbesondere zwischen 3,5 und 10% beträgt.

4. Pharmazeutische Zusammensetzungen gemäß einem der vorhergehenden Ansprüche, dadurch gekennzeichnet, daß der pH der Lösung auf einen Wert zwischen 4 und 7 und bevorzugt auf einen Wert von etwa 5,5 eingestellt ist.

5. Zusammensetzungen gemäß Anspruch 4, dadurch gekennzeichnet, daß, wenn die in Bezug auf das Dobutamin verwendete Menge an Ascorbinsäure zwischen 1 und 3 Gew.% beträgt, der pH bei etwa 5.5 festgelegt ist.

6. Pharmazeutische Zusammensetzungen gemäß einem der vorhergehenden Ansprüche, entsprechend der folgenden Formulierung:
| | |
|---|---|
| Dobutamin-Hydrochlorid | 280,2 mg |
| Na₂HPO₄ | 150 mg |
| Natriumascorbat | 27 mg |
| HCl 1N ausreichend für | pH 5.5 |
| Wasser ausreichend für | 20 ml |
